# EUROPEAN PATENT SPECIFICATION

(11) **EP 1 674 125 B1**
(45) Date of publication and mention of the grant of the patent: **29.04.2009**
(21) Application number: 06110626.6
(22) Date of filing: 16.08.1999
(51) Int. Cl.: A61M 25/01

(54) **A method of gastrostomy and an infection preventive cover and a gastrostomy catheter kit**
Gastrostomieverfahren und Infektionsschutzkappe für eine Kathetereinrichtung
Procédé pour la gastrostomie et gaine anti-infection pour un set de cathéter

(30) Priority: 17.08.1998 JP 23104198
(43) Date of publication of application: 28.06.2006
(62) Divisional of application: 99116044.1
(73) Proprietor: Suzuki, Yutaka, Funabashi-shi, 274-0825 Chiba (JP)
(72) Inventor: Suzuki, Yutaka, Funabashi-shi, 274-0825 Chiba (JP)
(74) Representative: Betten & Resch

(56) References cited:
- EP-A- 0 744 185
- WO-A-93/21984
- DE-A- 2 928 635
- GB-A- 1 601 691
- US-A- 3 894 540

## Description

### BACKGROUND OF THE INVENTION

### Field of the Invention

The present invention relates to a gastrostomy catheter kit as further disclosed in claim 1.

### Description of the Related Art

For a percutaneous endoscopic gastrostomy (PEG) enabling enteral feeding to a patient who finds difficulty in swallowing feeds or who cannot swallow, it is sufficient to apply a local anethesia to the patient and the operation time is favorably short, i.e., about five to about ten minutes, and the patient can be quickly recovered after the operation. In a case where the patient in a good general condition after the operation, she or he may leave the hospital on the same day on which the operation is conducted. The PEG is therefore explosively developed worldwide. In the United States, for example, about 180,000 cases were reported in 1997.
In the future, the number of the operations is expectedly increased in the world.

As commonly known, the PEG includes three methods, namely, "pull", "push", and "introducer" methods (techniques). Among these methods, the "pull" and "push" methods have been broadly adopted due to simplicity and safeness of the operation. However, these methods are attended with two drawbacks as follows.

The endoscope is required to be twice inserted in the pertinent patient, which leads to a problem of complex operations and pains to patients. There exists a fear of damage to the larynx, the upper pharynx or the esophagus.

The PEG catheter (including a PEG tube and a dome connected to the tube) is infected in the oral cavity, the upper pharynx or the larynx and hence the wounded part of the patient is liable to be infected.

The first drawback above can be sufficiently removed by improving the sedation or anesthesia and by increasing the quality of skill of the endoscopist. However, the second drawback, i.e., the infection of the wound due to the contamination of the PEG tube and the dome takes place with a high possibility. The literature of Europe and America reported about 35% to about 45% of the infection of wound. When the infection of wound occurs, antibiotics are required to be administered to the patient for a long period of time. This resultantly delays the starting point of the enteral feeding for the patient, and immunity of the patient from diseases is weakened, this may elongate the hospital treatment in some cases. The patient suffers from serious pains and the fee for medical treatment soars. Consequently, not only the patient but also family members of the patient must bear the expense and suffer from mental stress. When the cleaning of the oral cavity, the preoperation disinfection of the upper pharynx, and the preventive administration of antibiotics are completely carried out, the number of bacteria appearing on the PEG tube and the dome can be decreased. However, this is not the basic countermeasure.

### SUMMARY OF THE INVENTION

It is therefore an object of the present invention to provide a gastrostomy catheter (PEG catheter) kit to prevent the infection of the wound in a method of gastrostomy.

A method of gastrostomy including the steps of inserting a guide wire into a stomach through an outer tube pierced through a wall of an abdomen and a wall of the stomach of a patient and pulling the guide wire through an esophagus and an oral cavity into a space outside the patient, joining one end of a percutaneous endoscopic gastrostomy (PEG) catheter with the guide wire, delivering the PEG catheter into the stomach by pulling ("pulling method") or pushing ("pushing method") the guide wire, drawing the PEG catheter together with the outer tube through a hole in the wall of the stomach and the abdomen wall into a space outside the patient, keeping another end of the PEG catheter staying in the stomach (including a case in which the end is cut away from the catheter), is characterized by further comprising the steps of enclosing entirely the PEG catheter with an infection preventive cover having a sheath, the sheath having one opening end, closing the opening end of the sheath in the vicinity of the position of a joint between the PEG catheter and the guide wire, delivering the PEG catheter through the oral cavity into the stomach with the PEG catheter covered with the infection preventive cover, opening the opening end of the sheath in the stomach, drawing the PEG catheter into a space outside the patient, while drawing the PEG catheter through the opening end of the sheath opened, and removing thereafter the infection preventive cover from the oral cavity into a space outside the patient. The present invention is applicable to either the "push method" or the "push method" of gastrostomy.

The PEG catheter and the joint section between the PEG catheter and the guide wire are covered outside the oral cavity with the infection preventive cover having a sheath to be delivered from the oral cavity into the stomach in the covered state. Since the opening end of the infection preventive cover is closed until the cover reaches the stomach, the PEG catheter does not directly brought into contact with the oral cavity, the larynx and the upper pharynx of the patient. The PEG catheter is therefore kept clean.

The opening end of the infection preventive cover is opened in the stomach of the patient, and the PEG catheter is withdrawn through the opening end of the cover and is drawn through a hole (wounded part of the patient) prepared in the abdominal and stomach walls into a space outside the patient. When the PEG catheter is delivered into the stomach, the catheter is kept clean, namely, is not inflected. Even when the clean PEG catheter is brought into contact with the hole (wound) in the abdominal and stomach walls, the hole (wound) is hardly inflected. Consequently, infection of the wound can be efficiently prevented.

An infection preventive cover is used in the gastrostomy and comprises an elongated sheath having at least one opening end, a binding thread embroidered along a circumferential edge of the opening end of the sheath, and a cutting thread linked to the binding thread, the both ends of the binding thread being led outside the sheath at positions near to each other, and the cutting thread being stronger than the binding thread.

There can be considered various kinds of embroidering of the binding thread (a ligature) in the opening end of the sheath. For example, the ligature is reciprocally embroidered with an appropriate interval between an inner side and an outer side of the opening end portion of the sheath. Alternatively, the opening end portion of the sheath is folded back and the folded-back edge is fixed to the sheath by welding, melting or the like to thereby produce a threading bag, pass or guide, and the ligature is passed through the threading bag.

The cutting thread also includes a thread made of metal such as a metallic wire. Any chemical fiber such as nylon may be used if the cutting thread is stronger than the ligature. The sentence "a cutting thread is linked to the binding thread" includes a state in which the cutting thread is doubled to simply engage with the ligature.

For the infection preventive cover (the sheath), there is favorably employed thin, airtight, waterproof, flexible and strong materials such as vinyl and rubber. The PEG catheter may be covered with the infection preventive cover by inserting the PEG catheter into the elongated sheath.

Since the ligature is beforehand provided in the sheath, the operator need only bind the ligature to close the opening end of the sheath. Another method may also be adopted by use of the ligature to close the opening end. The operator need not be skillful with fingers to attach the sheath, and the gastrostomy can be hence completed in a short period of time.

The PEG catheter is entirely covered with the sheath of the infection preventive cover. Consequently, the infection of the wound can be prevented in the gastrostomy. The cutting thread can be drawn through the outer tube punctured into the abdomen wall or through the sheath into a space outside the patient. Consequently, by drawing the cutting thread to cut the ligature, it is possible to open the opening end of the sheath in the stomach.

An infection preventive cover kit includes an elongated sheath having at least one opening end, a binding thread (a ligature) to close the opening end of the sheath, and a cutting thread linked to the binding thread, the cutting thread being stronger than the binding thread.

The binding thread (a ligature) may be attached onto the sheath or may be separated therefrom. In either case, the opening end of the sheath covering the PEG catheter can be bound by the ligature into a closed state thereof. In the stomach, the ligature is cut by the cutting thread to thereby open the opening end of the sheath.

The present invention provides a PEG catheter kit utilizing the above infection preventive cover or the infection preventive cover kit. The PEG catheter kit comprises a PEG catheter, a sheath, into which the PEG catheter has been inserted, having at least one opening end, a binding thread to close the opening end of the sheath, and a cutting thread linked to the binding thread, the cutting thread being stronger than the binding thread. Since the PEG catheter has been covered with the sheath, a work for covering the PEG catheter with the sheath can be dispensed with. This also minimizes the period of time necessary for the gastrostomy. The opening end of the sheath covering the PEG catheter can be bound by the binding thread into a closed state thereof. By drawing the cutting thread, the binding thread is cut by the cutting thread to thereby open the opening end of the sheath in the stomach.

In one embodiment, the binding thread is embroidered along a circumferential edge of the opening end of the sheath, and the both ends of the binding thread are led outside the sheath at positions near to each other.

A PEG catheter kit in accordance with the present invention includes a PEG catheter, an elongated sheath entirety enclosing the PEG catheter, a cylindrical fixing device integrally provided to an end of the sheath, an end of the PEG catheter being arranged at the fixing device the fixing device having two junction surfaces adhered or melted to be closed, and a breaking wire for breaking a portion of the fixing device and a portion of the sheath the breaking wire being sandwiched between the two junction surfaces of the fixing device and adhered or melted on the inner surface of the sheath (1A).. Since the PEG catheter has been covered with the sheath, a work for covering the PEG catheter with the sheath can be dispensed with. This also minimizes the period of time necessary for the gastrostomy. Moreover, it may also be possible that the breaking thread is drawn through an outer tube pierced through the abdomen wall or through the sheath into a space outside the patient such that at least the fixing device (and favorably, a part of the sheath) is broken or cut away by the breaking thread in the stomach to open the opening end of the sheath so as to withdraw the PEG catheter out of the sheath.

Another PEG catheter kit comprises a PEG catheter, a sheath substantially enclosing the PEG catheter, an end of the sheath being closed, and a breaking thread, an end portion of which is fixed to the closed end portion of the sheath, for breaking the closed end portion of the sheath when the other end of the breaking thread is pulled. Since the PEG catheter has been covered with the sheath, a work for covering the PEG catheter with the sheath can be dispensed with. Moreover, it may also be possible that the breaking thread is drawn through an outer tube pierced through the abdomen wall or through the sheath into a space outside the patient such that a part of the sheath is cut away by the breaking thread in the stomach to open the opening end of the sheath so as to withdraw the PEG catheter out of the sheath.

In one embodiment, the catheter includes a joint wire extending from a top of the catheter, and the end of the sheath closes at a position of the joint wire. Preferably, a part of the joint wire which is exposed outside of the sheath is sufficiently disinfected before use.

### BRIEF DESCRIPTION OF THE DRAWINGS

The objects and features of the present invention will become more apparent from the consideration of the following detailed description taken in conjunction with the accompanying drawings in which:
Fig. 1 is a perspective view of an infection preventive cover;
Fig. 2 is a cross-sectional view schematically showing an upper half of a body of a patient in which a endoscope is inserted in the body in a PEG process;
Fig. 3 is a cross-sectional view schematically showing an enlarged part of a stomach of a patient in which a endoscope is inserted in the body in a PEG process;
Fig. 4 is a cross-sectional view schematically showing a part of a stomach of a patient in which a guide wire is inserted into the stomach in a PEG process;
Fig. 5 is a perspective view showing a linkage between a guide wire and a joint wire in a PEG process;
Fig. 6 is a cross-sectional diagram schematically showing a portion ranging from a head to an upper pharynx of a patient in a PEG process;
Fig. 7 is a cross-sectional view schematically showing an upper half of a body of a patient in which a PEG catheter covered with an infection preventive cover is pulled in a PEG process;
Fig. 8 is a cross-sectional view schematically showing an enlarged part of a stomach of a patient in which a PEG catheter covered with an infection preventive cover is pulled in a PEG process;
Fig. 9 is a cross-sectional view schematically showing an enlarged part of a stomach of a patient in which an opening end of a sheath is opened in a PEG process;
Fig. 10 is a cross-sectional view schematically showing an enlarged part of a stomach of a patient in which a PEG catheter is drawn out of a body in a PEG process;
Fig. 11 is a cross-sectional view schematically showing a part of a stomach of a patient in which a dome at an end of a PEG catheter abuts on a stomach wall in a PEG process;
Fig. 12 is a perspective view showing a state in which a PEG catheter is covered with an infection preventive cover;
Fig. 13 is an enlarged plan view schematically showing a state in which a cutting wire is fixed onto a guide wire;
Fig. 14 is a perspective view showing another example of the joint between a ligature at an opening end of an infection preventive cover and a cutting wire;
Fig. 15 is a side view showing a state of a PEG catheter covered with an infection preventive cover in another example in which a cutting wire bound on a ligature at an opening end of an infection preventive cover is drawn outwardly;
Fig. 16 is an enlarged perspective view showing another example in which a ligature is provided to an opening end of an infection preventive cover;
Fig. 17 is a cross-sectional view schematically showing an enlarged part of a stomach of a patient in a PEG process corresponding to Fig. 11 in which a one-step button is used;
Figs. 18A and 18B are perspective views showing another example of the ligaturing operation of an opening end of an inflection preventive cover;
Figs. 19A and 19B are perspective views showing further another example of the ligaturing operation of an opening end of an inflection preventive cover;
Figs. 20A to 20D collectively show a PEG catheter kit in which Fig. 20A is a partially cut-away perspective view of a PEG catheter kit, Fig. 20B is a cross-sectional view showing a state in which a joint wire is linked with a guide wire, Fig. 20C is a cross-sectional view showing a state in which the joint is moved into a sheath, and Fig. 20D is a perspective view showing a state in which a head and a sheath are partly broken; and
Figs. 21A to 21D are diagrams showing another example of a PEG catheter kit in which Fig. 21A is a side view of the PEG catheter kit, Fig. 21B is a partially cut-away side view showing a state in which a joint is covered with a slider, Fig. 21C is an enlarged cross-sectional view of a head along line C-C, and Fig. 21D is a cross-sectional view in a state in which the head is broken.
Fig. 22 is a partially cut-away perspective view showing still another example of a PEG catheter kit.

### DESCRIPTION OF THE PREFERRED EMBODIMENTS

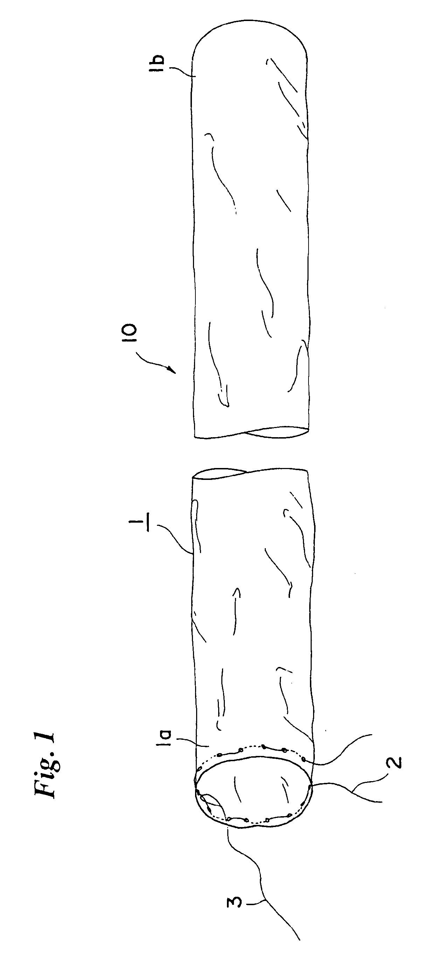

Fig. 1 shows an infection preventive cover used in a percutaneous endoscopic gastrostomy (PEG).

The infection preventive cover 10 includes an elongated sheath 1 having at least one end 1a opened. The sheath 1 includes another end 1b, which may be closed, opened, or provided with a hole. The sheath 1 has a length greater than that of a gastrostomy catheter (referred to as "a PEG catheter") 12, which will be described later. The diameter of the sheath 1 is substantially equal to, or greater than that of a dome 13 connected to an end edge of the catheter 12. When the sheath 1 is made of a elastic or expansive material, the diameter thereof may be slightly smaller than that of the dome 13. In either case, it is only required that the PEG catheter 12 including the dome 13 passes through the sheath 1. It is desirable that a lubricant such as a lubricant jerry is applied on an inner surface of the sheath 1.

The sheath 1 is manufactured with a thin, airtight, waterproof, flexible and strong material such as vinyl or a rubber. Favorably, the sheath 1 includes a possibly thin wall and has elasticity (especially in a circumferential direction). The sheath 1 is depicted in swollen out form to show a cylindrical and hollow body for easily understanding.

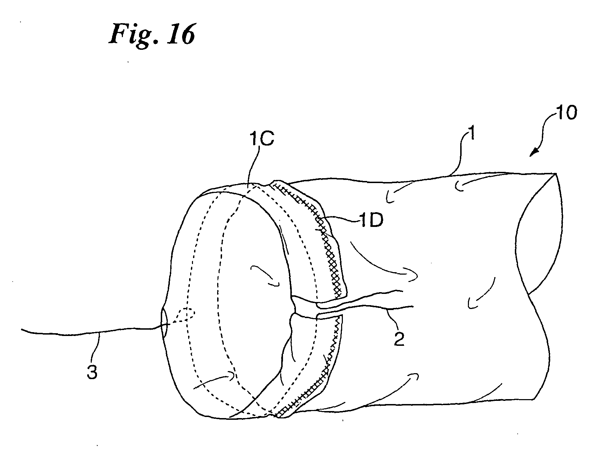

A circumferential periphery of the opening end 1a of the sheath 1 is embroidered with a ligature (a binding thread) 2 along the circumference, i.e., is continuously stitched from an outer side to an inner side and vice-versa with an appropriate interval (this state is referred to as "embroidered"). In addition to this state, the expression "embroidered with the ligature" also includes a state in which, as shown in Fig. 16, the circumferential periphery portion 1C of the opening end of the sheath 1 is folded back outside (or inside) and the folded-back portion 1C is melted (or welded or adhered) at its edge to the sheath 1 (the adhered portion is indicated by a reference numeral 1D) to form a bag, a path or guide along the edge of the opening end of the sheath 1, and the ligature 2 is passed therethrough. Both ends of the ligature 2 are outwardly led from the circumferential peripheral of the sheath 1 at positions near to each other. The ligature 2 is bound with one end of a cutting wire (thread) 3, which is guided out of the sheath 1. Favorably, the one end of the cutting wire 3 is coupled with the ligature 2 within the sheath 1. Furthermore, the cutting wire 3 is favorably coupled with the ligature 2 at a position most apart from the positions at which the ends of the ligature 2 are outwardly lead from the sheath 1 (i.e., the position of the cutting wire 3 and the positions of the ends of the ligature 2 are substantially opposing to each other on the sheath 1). In Fig. 16, the cutting wire 3 is led to outside through a hole formed on the bag, path or guide.

The cutting wire 3 is stronger than the ligature 2. For example, a thin metallic thread is employed as the cutting wire and a silk thread is utilized as the ligature 2. For the cutting wire 3 and the ligature 2, there may be employed threads respectively made of linen, cotton, polyester, polyethylene, and any other vegetable or chemical fiber.

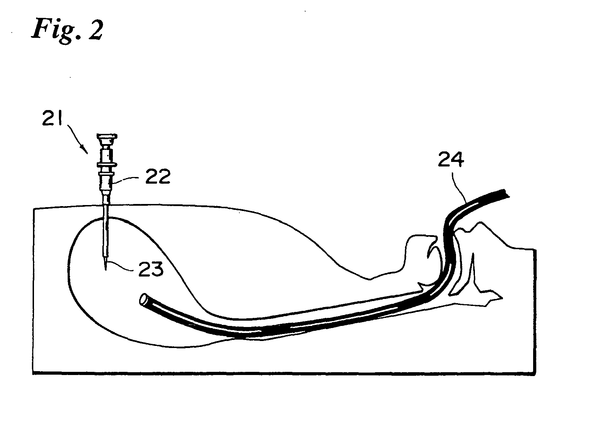

Referring now to Figs. 2 to 13, description will be given in detail of a usage method and a role of the infection preventive cover 10 configured above in relation to the PEG method. In this example, a method called "pull method (technique)" will be described. The PEG method is performed in general by an operator, an endoscopist and one or two nurses.

An endoscope 24 is inserted from a mouth of a patient in a supine position into her or his stomach. Air is fed through the endoscope 24 into the stomach of the patient to expand the stomach to resultantly tightly fix the stomach wall onto a peritoneum of the patient. A puncturing part is determined and its peripheral is completely disinfected. After the periphery is locally anesthetized, about one centimeter of skin is incised in the puncturing part and then a needle 21 with an outer tube is pierced thereinto (Fig. 2).

The needle 21 includes an outer tube (pipe) 22 of a cylindrical contour and a needle (inner tube 23) having a sharp end. The outer tube 22 is hallow. With the needle 23 completely installed in the external tube 22, the sharp end of the needle 23 is projected from an end of the outer tube 22. The sharp end of the needle 23 thrusts into the abdomen wall, the peritoneum and the stomach wall, and the outer tube 22 also passes through the abdomen wall, the peritoneum and the stomach wall.

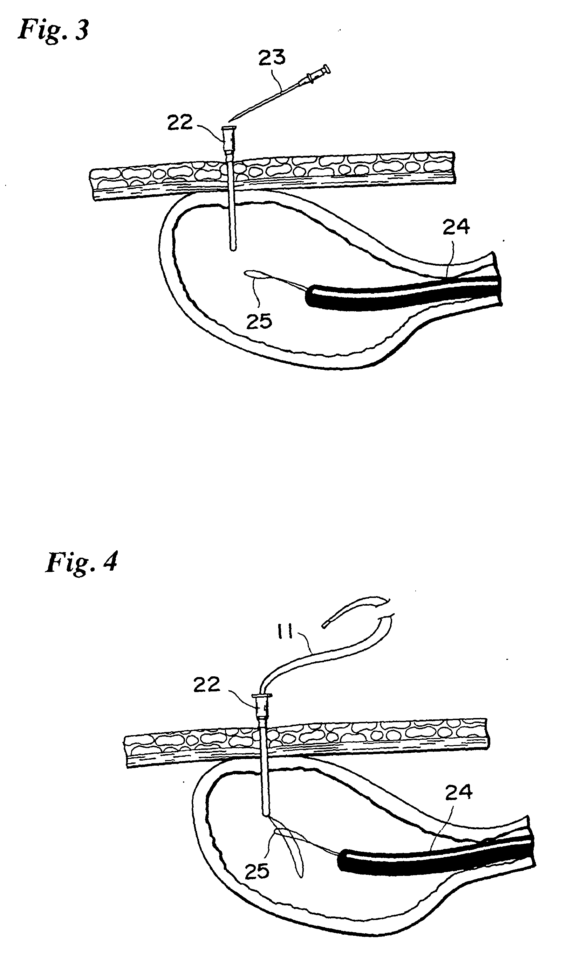

The needle 23 is removed from the outer tube 22. The outer tube 22 is kept pierced ranging from the abdomen wall to the stomach wall. An end of a snare forceps 25 is drawn from an end of the endoscope 24 to be exposed in the stomach (Fig. 3).

A guide wire 11 is passed through the outer tube 22 to be inserted into the stomach (Fig. 4).

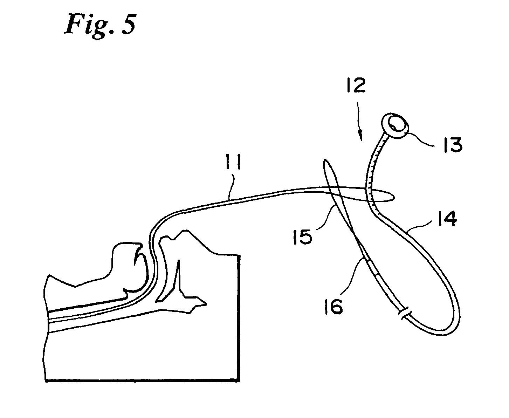

An end of the guide wire 11 fed into the stomach is grasped by he snare forceps 25. The guide wire 11 held by the snare forceps 25 is withdrawn out of the oral cavity of the patient together with the endoscope 24. After the guide wire 11 is sufficiently drawn out of the oral cavity, the snare forceps 25 is released from the guide wire 11. The guide wire 11 thus drawn out of the oral cavity is coupled with a joint wire 15 of the PEG catheter 12 (Fig. 5).

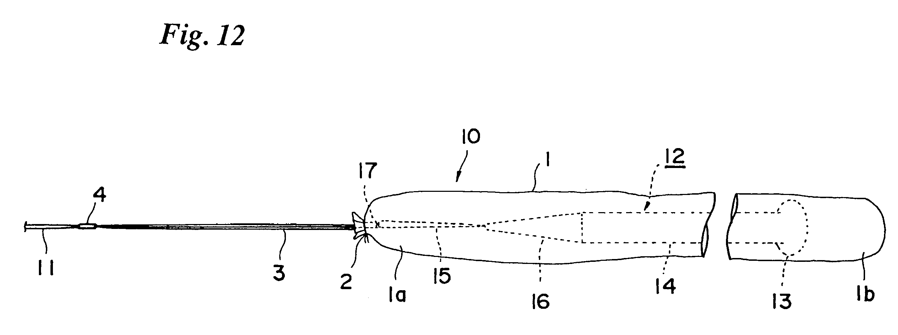

The PEG catheter 12 includes a PEG tube 14 which has one end at which a dome 13 is integrally coupled with or connected to and which has another end tapered in a cone shape (this section is called a taper section 16). The taper section 16 is linked with the joint wire 15. A end of the joint wire 11 in a doubled form is entangled with the joint wire 15 such that the guide wire 11 is coupled with the joint wire 15 (the section of the coupling between the guide wire 11 and the joint wire 15 is called a joint 17; reference is also to be made to Fig. 12).

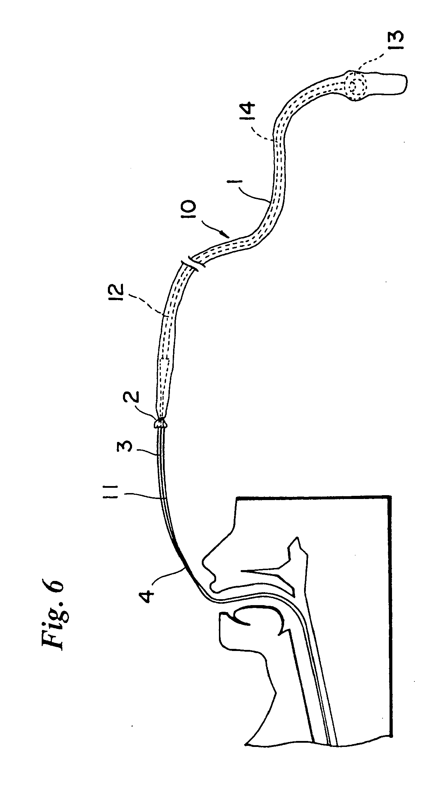

Thereafter, a portion of the PEG catheter 12 ranging from the dome 13 to the joint 17 is inserted into the sheath 1 of the infection preventive cover 10 and then both ends of the ligature 2 are tightly fastened (Fig. 6).

Fig. 12 shows in an enlarged diagram a state in which the opening end 1a of the sheath 1 of the infection preventive cover 10 is tied up by the ligature 2. The ligature 2 is tightly bound, for example, in a surgical knot at a position slightly shifted from the joint 17 in the sheath 1 toward the side of the guide wire 11. As a result, the opening end 1a of the sheath 1 is fixedly tightened to be closed (the opening end 1a thus ligatured of the sheath 1 will be referred to as a ligatured end herebelow). An unnecessary section of each end of the ligature 2 is cut away.

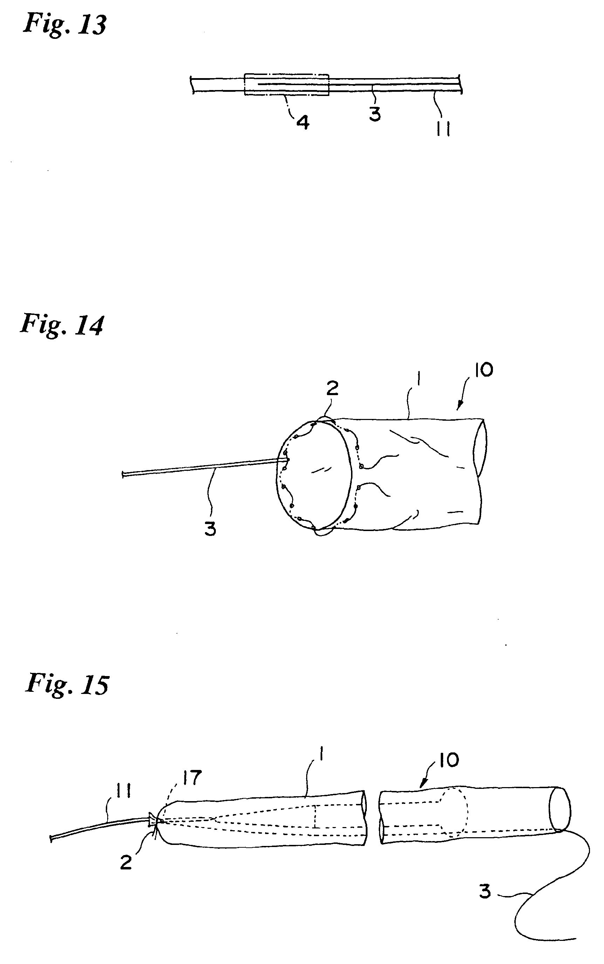

The cutting wire 3 is extended along the guide wire 11 and an end of the cutting wire 3 is fixed onto the guide wire 11 by a fixing unit such as a tape 4. Fig. 13 schematically shows an enlarged image of the end of the cutting wire 3 fixed onto the guide wire 11. The fixed section (tape 4) of the cutting wire 3 must be of a small size to pass through the outer tube 22.

As can be seen from Fig. 14, it may also be possible that the ligature 2 is engaged with or hooked by the cutting wire 3, and the cutting wire 3 is bent (without being bound), the cutting wire 3 in the double form is arranged along the guide wire 11, and then both ends of the cutting wire 3 are fixed onto the guide wire 11.

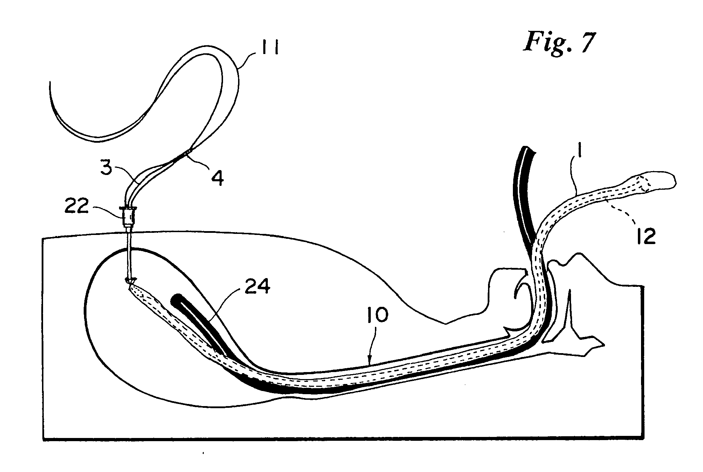

The end of the guide wire 11 drawn through the outer tube 22 into a space outside the body of the patient is pulled. This causes the PEG catheter 12 coupled with the guide wire 11 to be delivered through the oral cavity, the upper pharynx and the larynx into the stomach with the catheter 12 covered with the sheath 1 (Fig. 7).

At the same time, the endoscope 24 is again inserted through the oral cavity into the stomach. By inserting the endoscope 24 along the PEG catheter, it is possible to smoothly move the endoscope 24 into the stomach of the patient.

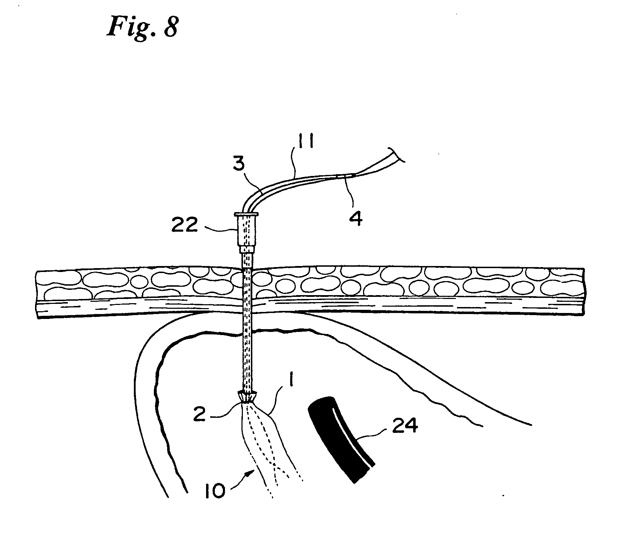

The cutting wire 3 fixed onto the guide wire 11 is also withdrawn together with the guide wire 11 through the outer tube 22 out of the abdomen wall. When the guide wire 11 is further drawn, the ligatured end of the sheath 1 abuts on an end of the outer tube 22 (Fig. 8). It is favorable to confirm this event, i.e., the ligatured end of the sheath 1 abuts on an end of the outer tube 22 by the endoscope 24. In this state, the PEG catheter 12 and another end of the sheath 1 are still outside the mouth of the patient (Fig. 7). It may also be favorable to confirm by a hand that the ligatured end of the sheath 1 abuts on the end of the outer tube 22. It may thereafter be possible to insert the endoscope 24 into the stomach. The second insertion of the endoscope 24 may be avoided.

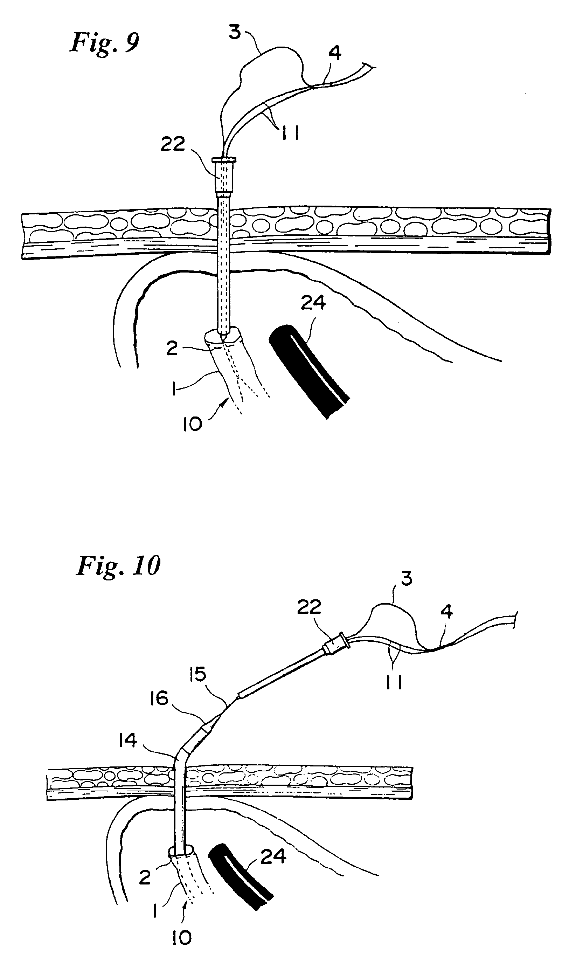

The cutting wire 3 outside the patient is then drawn (pulled). In this situation, it is favorable that the endoscopist holds the sections of the PEG catheter 12 and the sheath 1 outside the mouth of the patient by slightly pulling these sections. Since the cutting wire 3 is stronger than the ligature 2, the ligature 2 is cut by the cutting wire 3 in the stomach. Resultantly, the ligatured state of the sheath 1 by the ligature 2 is released and the opening end of the sheath 1 is opened (Fig. 9). If necessary, a section of the ligature 2 is fixed onto the sheath 1 by an adhesive or the like such that the ligature 2 thus cut off does not fall into the stomach.

It is desirable that the opening end 1a of the sheath 1 is beforehand prepared to easily open outwardly, for example, by bending the opening end 1a several times or by giving nature to open. With this preparation, it is guaranteed that the sheath 1 opens when the ligature 2 is cut off.

It may also be possible that the cutting wire 3 is outwardly withdrawn on the side of the oral cavity of the patient to thereby cut off the ligature 2. In this case, as shown in Fig. 15, an end of the cutting wire 3 is linked with the ligature 2 in the sheath 1 and another end thereof is passed through the sheath 1 to be outwardly fed through an opening (or a hole) on another end of the sheath. When the cutting wire 3 is pulled on the side of the oral cavity, the ligature 2 is cut off. If a section of the cutting wire 3 near the ligatured end is adhered, welded or melted onto an inside of the sheath 1, the sheath 1 is also broken up to an intermediate part thereof to provide a larger opening when the cutting wire 3 is pulled and the ligature 2 is accordingly cut off.

While the outer tube 22 is being drawn through the stomach and abdomen walls, the guide wire 11 is further withdrawn outwardly. The joint wire 15, the taper section 16, and the PEG tube 14 are delivered through the stomach wall and the abdomen wall into a space outside the patient body (Fig. 10).

When the PEG catheter 12 is being drawn toward the outside of the patient body, the endoscopist holds by a hand the end 1b of the sheath 1 outside the mouth of the patient such that the sheath 1 is not fed into the patient body.

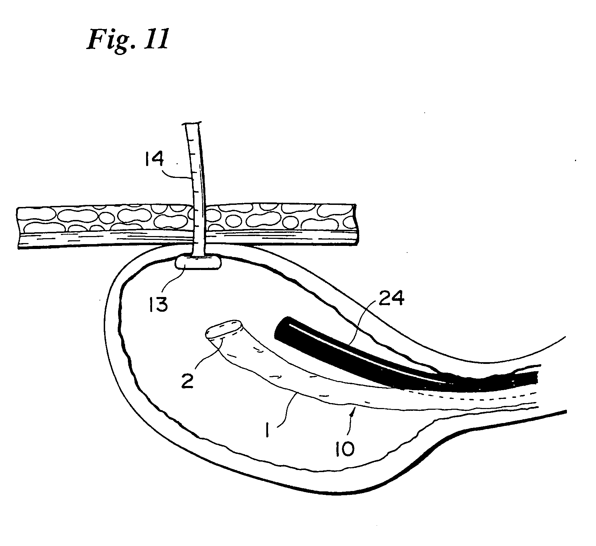

Finally, the dome 13 appears from the opening end of the sheath 1 and abuts on the stomach wall (Fig. 11). If necessary, this condition that the dome 13 abuts on the stomach wall is confirmed by the endoscope 24. The sheath 1 is removed from the mouth of the patient into a space outside the patient.

The PEG tube 14 thus withdrawn is cut at an appropriate point to have a necessary length, and the cut-off end is connected with an adapter to supply a medicine for nutrition. The PEG tube is attached onto the body of the patient with an appropriate fixing unit (means), thereby completing the operation of the PEG method.

Outer surfaces of the guide wire 11 and the sheath 1 having passed through the larynx, the upper pharynx and the oral cavity are infected by bacteria on the oral cavity, the upper pharynx and the larynx. However, since the guide wire 11 is drawn through the outer tube 22 into a space outside the patient body, it hardly occurs that the wound (hole) in the stomach and abdomen walls is infected by the guide wire 11. Furthermore, the joint 17 between the guide wire 11 and the joint wire 15, the joint wire 15, the taper section 16, the PEG tube 14 and the dome 13 are each covered with the sheath 1 to be fed, in this state, through the oral cavity, the upper pharynx and the larynx into the stomach to be then withdrawn from the sheath 1 in the stomach. Even when the joint 17, the joint wire 15, the taper section 16 and the PEG tube 14 are brought into contact with the wound when they are drawn to a space outside the patient, there is almost no chance that the wound is contaminated by bacteria. The sheath 1 of which outer surfaces are infected are removed through the mouth of the patient. It does not occur that the wound is infected by the infection preventive cover 10. In consequence, the infection of the wound can be advantageously prevented.

Also in the "push" method, it is possible to effectively prevent infection of the wound by pushing the PEG catheter 12 covered with the infection preventive cover 10 into the stomach.

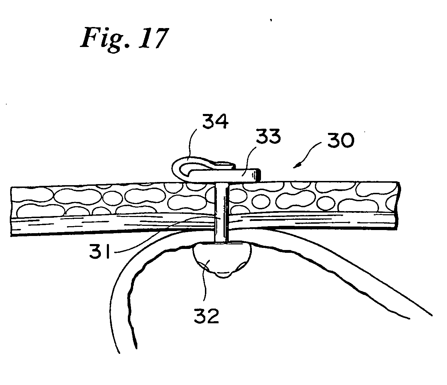

Fig. 17 partly shows a PEG catheter (PEG tube) of a button type, the button being called one-step button. The button 30 is attached onto a tip end of a PEG tube of the PEG catheter. When the PEG tube is sufficiently drawn from the stomach into an external space of the body (corresponding to the state of Fig. 11), the button 30 is separated from the PEG tube. The one-step button 30 includes a shaft 31; a dome 32 including a counterflow preventive valve and integrally connected to an end of the shaft 31, the dome 32 being kept remained in the stomach; a stopper 33 formed integral with the shaft 31, the stopper 32 abuting on the outside of the abdomen wall; and a cap 34 linked to the stopper 33 to seal a hallow section of the shaft 11. The infection preventive cover 10 can be applied also to the button-type PEG catheter including the one-step button. It is also to be understood that the infection preventive cover 10 is applicable to PEG catheters in another configuration.

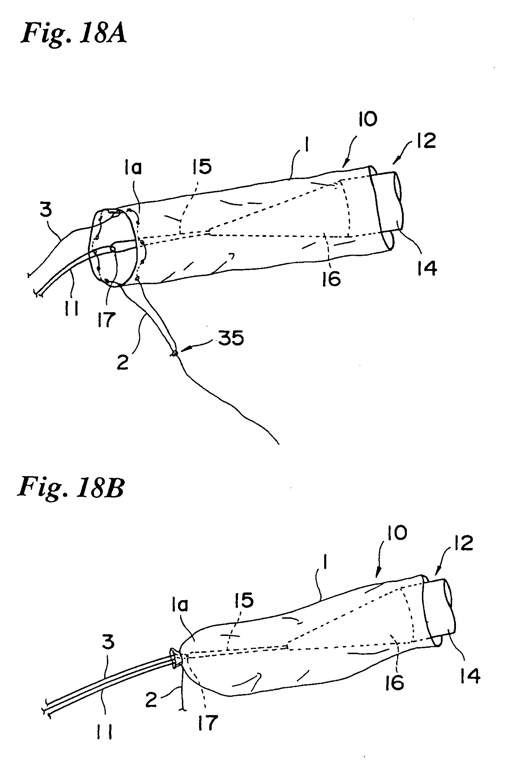

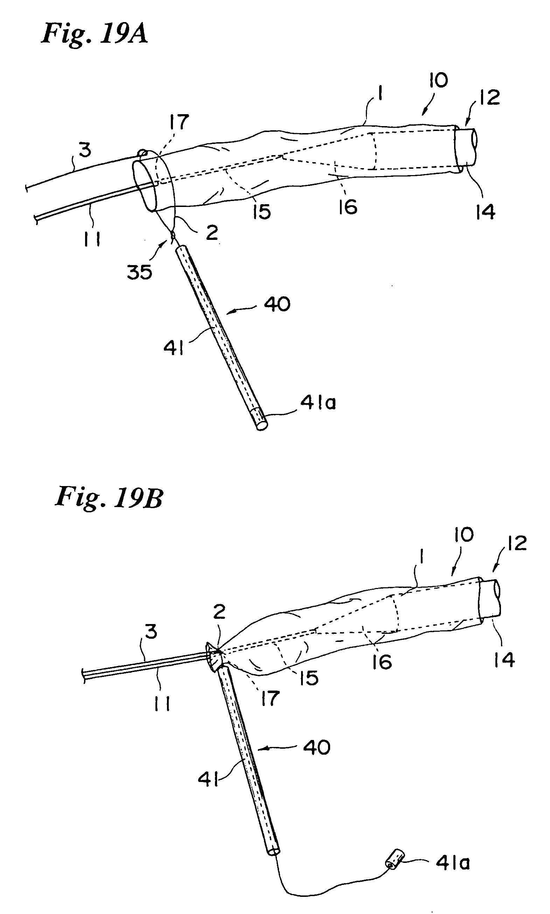

Figs. 18A, 18B, 19A, and 19B show further examples of the ligature at the opening end of the infection preventive cover. In these diagrams, the same components as those of Fig. 1 are assigned with the same reference numerals and duplicated description thereof will be avoided.

Referring to Figs. 18A and 18B, the circumferential periphery of the opening end 1a of the sheath 1 is beforehand embroidered with the ligature 2. At an intermediate point of an end section of the ligature 2, another end thereof is fastened by a roller knot 35 (Fig. 17A). The ligature 2 is coupled with the cutting wire 3. The operator connect the guide wire 11 to the joint wire 15, covers the PEG catheter 12 with the sheath 1, and then pulls the one end of the ligature 2. The opening end 1a of the sheath 1 can be quite simply tightened (Fig. 17B). An unnecessary end section of the ligature 2 is to be cut away.

In Figs. 19A and 19B, a ligaturing unit 40 is employed. The ligaturing unit 40 includes a ligature 2 and a tightening rod 41. In the tightening rod 41, a hole is provided along a longitudinal direction thereof. The tightening rod 41 includes a cut-away groove at an end portion thereof. The end portion of the rod 41 up to the cut-away groove is indicated by a reference numeral 41a. The ligature 2 is formed in a loop and an end section thereof is passed through the hole of the tightening rod 41 to be fixedly attached to the end portion 41a. Another end of the ligature 2 is fastened at an intermediate point of the ligature 2 by a roller knot 35. The loop of the ligature 2 is linked with the cutting wire 3.

The operator puts the sheath 1 on the PEG catheter 12 coupled with the guide wire 11, passes the PEG catheter 11 and the sheath 1 through the loop of the ligature 2, and places the loop section of the ligature 2 at a position of the sheath 1, the position being slightly apart from the opening end 1a (Fig. 19A). The operator bends or cuts away the end portion 41a of the tightening rod 41 and pulls the end portion 41a. The loop of the ligature 2 is tightened to close the opening circumferential periphery of the sheath 1 and the closed state is kept retained (Fig. 19B). An unnecessary part of the ligature 2 is cut away. It may naturally be possible that the opening circumferential periphery of the sheath 1 is embroidered with the ligature 2.

A PEG catheter kit can be provided. The PEG catheter kit includes a combination of a PEG catheter and the above mentioned infection preventive cover, that is, a PEG catheter covered with the infection preventive cover. A work for inserting a PEG catheter into a sheath of the infection preventive cover can be dispensed with.

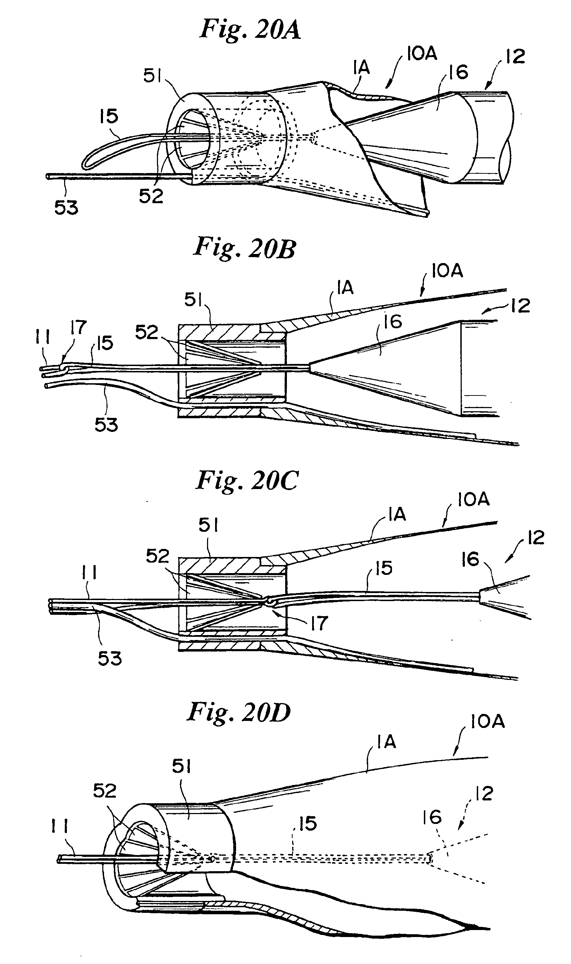

Figs. 20A and 20D show another PEG catheter kit in which a PEG catheter and an infection preventive cover are beforehand combined.

Referring particularly to Figs. 20A and 20B, the infection preventive cover 10A includes a head 51 and a sheath 1A.

The head 51 has a cylindrical contour and includes in its hole section a plurality of movement preventive pieces 52. The sheath 1A is fabricated in a tapered form and a tapered edge section thereof has a slightly thicker wall. Naturally, the sheath 1A may be of a uniform wall thickness. The head 51 includes a step section at which the tapered end section of the sheath 1A is fixed (welded or adhered).

The PEG catheter 12 is beforehand installed in the sheath 1A and the joint wire 15 of the catheter 12 is outwardly guided through the hole of the head 51. The movement preventive pieces 52 of the head 51 are constructed to be aligned inwardly from the end edge section of the head 51 toward the sheath 1A.

The head 51 is longitudinally cut off at a position thereof to form two junction surfaces, between which a breaking wire 53 is inserted. In this state, the junction surfaces of the head 51 is adhered or melted to be closed, sandwiching the breaking wire 53. One end section of the breaking wire 53 extends along an inner surface of the sheath 1A up to an intermediate point thereof and is adhered or melted on the inner surface of the sheath 1A. Another end section of the breaking wire 53 is lead to the outside of the sheath 1A and the head 51. It may also be possible that the section of the sheath 1A in which the breaking wire 53 is adhered or melted may also be cut off such that the cut-off section is thereafter adhered or melted together with the breaking wire 53.

As in the embodiment described above, the guide wire 11 is drawn out of the mouth of the patient and then is coupled with the joint wire 15 (Fig. 20B).

The head 51 is moved along the joint wire 15 to move the joint 17 into the sheath 1A. The joint 17 is brought into contact with ends of the movement preventive pieces 52 and hence the head 51 is prevented from moving in a direction of the joint wire 15. The breaking wire 53 is aligned with the guide wire 11 and an end section thereof is fixed on the guide wire 11 by a fixing tape or the like in almost the same way as for the cutting wire 3 shown in Fig. 13 (Fig. 20C).

By drawing the guide wire 11 through the outer tube 22 pierced through the wall of the abdomen of the patient, the PEG catheter 12 is delivered together with the infection preventive cover 10A through the mouth, the upper pharynx, the larynx and the esophagus to the stomach. When the head 51 abuts on an inner end of the outer tube 22, the breaking wire 53 which is exposed outside together with the guide wire 11 are pulled. Part of the head 51 and part of the sheath 1A are resultantly broken (Fig. 20D).

In this state, while continuously holding the end edge section of the sheath 1A outside the mouth of the patient, the operator draws the PEG catheter 12 together with the outer tube 22 out of the abdomen of the patient.

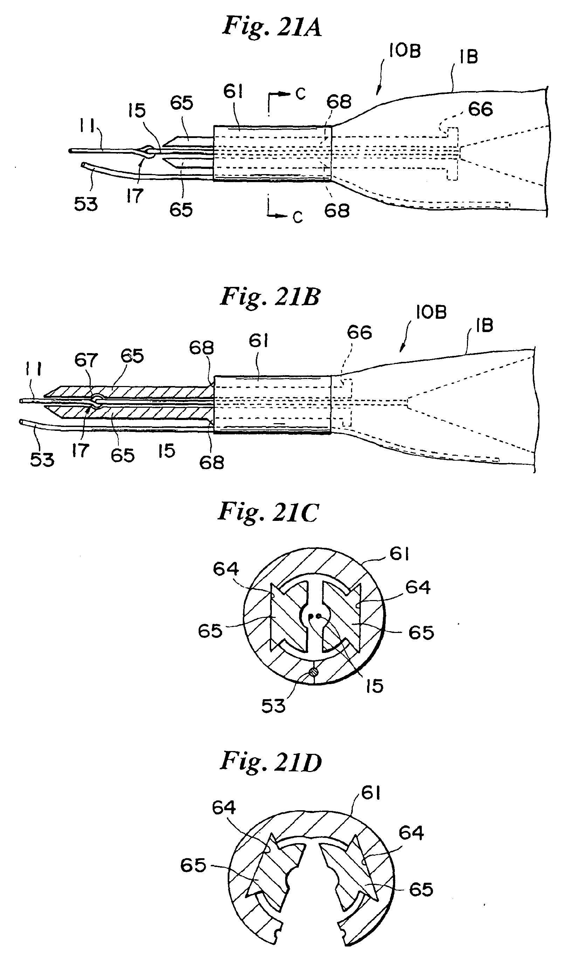

Figs. 21A to 21D still show another example of a PEG catheter kit.

The infection preventive cover 10B includes a head 61 and a sheath 1B. The head 61 is fixed on a tip end of the sheath 1B and a breaking wire 53 is arranged as described in the embodiment above.

On an inner circumferential surface of the head 61 having a cylindrical shape, two guide grooves (dovetail grooves) 64 are formed, the grooves opposing each other. On each guide groove 64 is engaged a slider (dovetail) 65 to move in an axial direction of the head 61 (Fig. 21C). The slider 65 includes an end section on the side of the sheath 1B, the end section including a stopper 66. At an intermediate section of the slider 65, there is formed a cut-away section 68 to form a section serving as a stopper 68. On an inner surface of a tip end section of the slider 65, there is formed a depression 67.

When the slider 65 is pulled outwardly, the joint 17 engages in the depression 67 of the slider 65 and the cut-away section of the slider 65 is brought into contact with the outer end section of the head 61 to function as the stopper 68 (Fig. 21B).

If the operator pulls the breaking wire 53 when the head 61 reaches the stomach of the patient, part of the head 61 and part of the sheath 1B are broken (Fig. 21D).

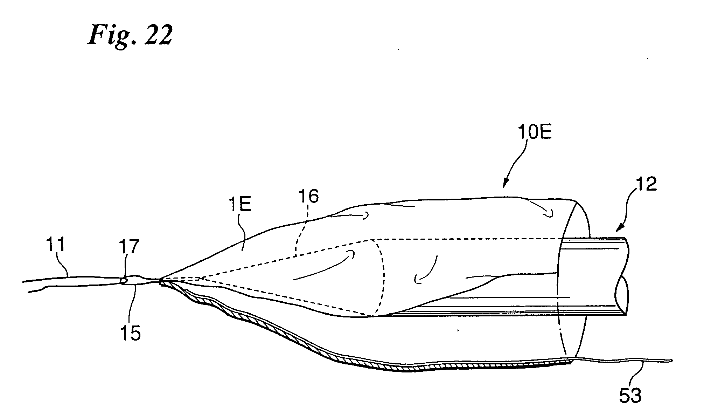

Fig. 22 shows further example of a PEG catheter kit. An infection preventive cover 10E comprises a sheath 1E and a breaking wire 53. The top of the sheath 1E is tapered to be closed at a position of the joint wire 15 extended from the taper section 16 of the PEG catheter 12. A part of the joint wire 15 is outside the sheath 1E, so that the joint wire 15 can be coupled with the guide wire 11. Preferably the exposed portion of the point wire 15 is sufficiently disinfected.

A breaking wire 53 extends to the top portion of the sheath 1E along the inner surface of the sheath 1E to be adhered or melted to the inner surface of the sheath 1E. Preferably, a portion of the sheath 1E, on which the breaking wire 53 is adhered, is weakened. For example, a part of the sheath 1E is cut, the breaking wire 53 is adhered to along the cut lines, and the cut lines of the sheath 1E is melted to be adhered to each other together with the breaking wire 53. The other end of the breaking wire 53 is led outside of the sheath 1E.

When the top (the taper section 16) of the PEG catheter 12 covered with the sheath 1E reaches the stomach of the patient, if the operator pulls the braking wire 53 in the side of the mouth of the patient, the top portion of the sheath 1E is broken.

While the present invention has been described with reference to the particular illustrative embodiments, it is not to be restricted by those embodiments,

It is to be appreciated that those skilled in the art can change or modify the embodiments without departing from the scope of the present invention.

## Claims

1. A percutaneous endoscopic gastrostomy catheter kit, comprising:
a percutaneous endoscopic gastrostomy catheter (12);
a sheath (1A) entirely enclosing the catheter (12);
a cylindrical fixing device (51) integrally provided to an end of the sheath (1A) an end of the catheter being arranged at the fixing device, the fixing device having two junction surfaces adhered or melted to be closed,
a breaking wire (53) for breaking a portion of the fixing device and a portion of the sheath (1A), the breaking wire being sandwiched between the two junction surfaces of the fixing device and adhered or melted on the inner surface of the sheath (1A).

## Patentansprüche

1. Perkutanendoskopie-Gastrostomiekathetersatz, umfassend:
einen Perkutanendoskopie-Gastrostomiekatheter (12);
eine Hülle (1A), die den Katheter (12) vollständig umschließt;
eine zylindrische Befestigungsvorrichtung (51), die integral an einem Ende der Hülle (1A) vorgesehen ist, wobei ein Ende des Katheters an der Befestigungsvorrichtung angeordnet ist, wobei die Befestigungsvorrichtung zwei Verbindungsflächen aufweist, die verklebt oder verschmolzen sind, um geschlossen zu sein,
einen Bruchfaden (53) zum Brechen eines Bereichs der Befestigungsvorrichtung und eines Bereichs der Hülle (1A), wobei der Bruchfaden zwischen die zwei Verbindungsflächen der Befestigungsvorrichtung eingelegt und an die innere Fläche der Hülle (1A) geklebt oder geschmolzen ist.

## Revendications

1. Kit de cathéter de gastrostomie endoscopique percutanée, comprenant :
un cathéter de gastrostomie endoscopique percutanée (12) ;
une gaine (1A) qui recouvre totalement le cathéter (12) ;
un dispositif de fixation cylindrique (51) intégralement prévu sur une extrémité de la gaine (1A), une extrémité du cathéter étant disposée au niveau du dispositif de fixation, le dispositif de fixation ayant deux surfaces de jonction collées ou fondues pour être fermées,
un fil métallique de rupture (53) permettant de rompre une partie du dispositif de fixation et une partie de la gaine (1A), le fil métallique de rupture étant pris en sandwich entre les deux surfaces de jonction du dispositif de fixation et collé ou fondu sur la surface interne de la gaine (1A).
